# EUROPEAN PATENT SPECIFICATION

(11) **EP 3 815 547 B1**
(45) Date of publication and mention of the grant of the patent: **17.07.2024**
(21) Application number: 18924346.2
(22) Date of filing: 17.09.2018
(51) Int. Cl.: A23L 33/165, A23L 33/175, A61K 31/198, A61K 31/4172, A61K 33/30, A61K 47/64, A61P 35/00

(54) **ZINC-CONTAINING, WATER-SOLUBLE POLYGLUTAMIC ACID COMPLEX COMPOSITION**
ZINKHALTIGE WASSERLÖSLICHE POLYGLUTAMINSÄUREKOMPLEXZUSAMMENSETZUNG
COMPOSITION COMPLEXE D'ACIDE POLYGLUTAMIQUE, SOLUBLE DANS L'EAU, CONTENANT DU ZINC

(30) Priority: 28.06.2018 KR 20180074477
(43) Date of publication of application: 05.05.2021
(73) Proprietor: Alextand Co., Ltd., Gangnam-gu, Seoul 06376 (KR)
(72) Inventor: BAE, Jin Ho, Seoul 06366 (KR)
(74) Representative: WSL Patentanwälte Partnerschaft mbB
(86) International application number: PCT/KR2018/010881
(87) International publication number: WO 2020/004712

(56) References cited:
- EP-A1- 1 723 855
- WO-A1-2007/043606
- WO-A1-2007/043606
- WO-A1-2012/039518
- WO-A1-2018/084806
- JP-A- 2011 062 164
- JP-A- H0 578 243
- KR-A- 20120 094 546
- US-A1- 2018 110 731
- US-B1- 7 863 258
- SUBARNA KARMAKER ET AL: "A Zinc(II)/Poly( γ -glutamic acid) Complex as an Oral Therapeutic for the Treatment of Type-2 Diabetic KKA y Mice", MACROMOLECULAR BIOSCIENCE, vol. 9, no. 3, 10 March 2009 (2009-03-10), DE, pages 279 - 286, XP055439227, ISSN: 1616-5187, DOI: 10.1002/mabi.200800190
- SCHOLMERICH, JURGEN ET AL.: "Bioavailability of zinc from zinc-histidine complexes. 1. Comparison with zinc sulfate in healthy men", THE AMERICAN JOURNAL OF CLINICAL NUTRITION, vol. 45, 1987, pages 1480 - 1486, XP055666955

## Description

### [Technical Field]

The present invention relates to a zinc-containing water-soluble polyglutamic acid complex composition and to the use thereof.

### [Background Art]

Zinc, which is essential for human body to realize homeostasis and health, has been recently supplied in the form of zinc oxide, which is not readily soluble in water, and thus the absorption rate thereof into the body is very low.

Therefore, there have been studies to increase the rate of absorption of zinc into the body. SSV Therapeutics Inc., in the United States granted a patent in 2012 relating to mitigation of copper toxicity by preparing a complex of general amino acid and zinc (Om P. Goel, US 8,247,398 B2 (August 21, 2012). Tami et al., of the University of Bern in Switzerland published the results of use of a complex of zinc and general amino acids prepared using zinc acetate as a catalyst for aldol condensation (Tamis Darbre et al., Chem. Comm. 2003, 1090-1091). Somaya et al., in Iran reported that a complex of zinc and common amino acids remains in the soil for much longer than the case of the use of amino acids alone in plants (Somayeh Ghasemi et al., Soil Biology & Biochemistry. 2013, 63, 73-79) . Ferrer et al., published a method of preparing a zinc-histidine complex using zinc carbonate (P. Ferrer et al., Journal of Physical Chemistry, 2014, 118, 2842-2850).

### [Prior Art document]

### [Patent Document]

(Patent Document 1) US Patent No. 8,247,398 B2
(Patent Document 2) WO 2007/043606 A1 discloses a combination of polygamma glutamic acid and a metal compound, such as a zinc compound.
(Patent Document 3) US 2018/110731 A1 discloses the use of a complex comprising polyglutamic acid and zinc in the preparation of a drug delivery system.
(Patent Document 4) WO 2018/084806 A1 discloses pharmaceutical compositions comprising a polyglutamic acid carrier and a zinc salt.
(Patent Document 5) US 7 863 258 B1 discloses nanoparticles including polygamma glutamic acid.
(Patent Document 6) WO 2012/039518 A1 discloses compositions comprising zinc.
(Patent Document 7) JP H05 78243 A is directed to the synthesis of zinc complexes, where the ligands are amino acids.

### [Non-Patent Document]

(Non-Patent Document 1) Tamis Darbre et al., Chem. Comm. 2003, 1090-1091.
(Non-Patent Document 2) Somayeh Ghasemi et al., Soil Biology & Biochemistry. 2013, 63, 73-79.
(Non-Patent Document 3) P. Ferrer et al., Journal of Physical Chemistry, 2014, 118, 2842-2850.
(Non-Patent Document 4) Subarna Karmaker et al., Macromolecular Bioscience, 2009, vol. 9, no. 3, p. 279-286, disclose a zinc/polygamma glutamic acid complex as an oral therapeutic for the treatment of type-2 diabetic mice.

### [Disclosure]

### [Technical Problem]

As described above, zinc, which is essential for human body to realize homeostasis and health, has recently been supplied in the form of zinc oxide, which is not readily soluble in water, and thus the absorption rate thereof into the body is very low. Accordingly, the present inventors prepared a novel material of a zinc-chelated complex by reacting histidine and polyglutamic acid with zinc while conducting research on increasing the absorption rate of zinc, and identified that the novel material is highly soluble in water. In addition, the present inventors identified that the novel material has significant anticancer activity and is effective in relieving pollinosis resulting from irritation of the mucous membrane by pollen.

Therefore, it is one object of the present invention to provide a stable zinc-polyglutamic acid complex (Alex2) that is highly soluble in water by binding zinc to histidine and polyglutamic acid.

It is another object of the present invention to provide a capsule of the complex as a supply of zinc essential for the human body.

It is another object of the present invention to provide a capsule of the complex having anticancer activity and an effect of relieving pollinosis resulting from irritation of the mucosa membrane by pollen.

It is another object of the present invention to provide an injectable formulation of the complex as a supply of zinc essential for the human body.

It is another object of the present invention to provide an injectable formulation of the complex having anticancer activity and an effect of relieving pollinosis resulting from irritation of the mucosa membrane by pollen.

The objects of the present invention are not limited to those described above. The objects of the present invention will be clearly understood from the following description and may be implemented by the means defined in the claims and combinations thereof.

### [Technical Solution]

The present invention provides a zinc//histidine/polyglutamic acid complex in accordance with claim 1.

The inventive complex is prepared from 30 to 70 parts by weight of the polygamma glutamic acid and 30 to 150 parts by weight of the zinc supply based on 200 parts by weight of the histidine.

In another aspect of the present invention, the zinc supply includes at least one of zinc oxide, zinc acetate and zinc carbonate.

In another aspect of the present invention, the complex contains zinc in an amount of 15% by weight or more based on the total weight of the complex.

In one aspect of the present invention, the complex is prepared by adding histidine, polygamma glutamic acid and a zinc supply to water, followed by reflux and then precipitation using a lower alcohol having 1 to 5 carbon atoms.

In another aspect, the present invention provides a method for preparing a zinc/histidine/polyglutamic acid complex, including:
(a) adding histidine, polygamma glutamic acid, and a zinc supply to water, followed by reflux;
(b) distilling the refluxed mixture; and
(c) adding a lower alcohol having 1 to 5 carbon atoms thereto to precipitate the complex.

According to the present invention, the step (a) includes adding 30 to 70 parts by weight of the polygamma glutamic acid and 30 to 150 parts by weight of the zinc supply, based on 200 parts by weight of the histidine, followed by reflux.

In another aspect of the present invention, the reflux is carried out for 30 minutes to 4 hours, and the lower alcohol having 1 to 5 carbon atoms includes at least one of isopropanol, butanol and ethanol.

In another aspect, the present invention provides a health food composition for supplying zinc containing the zinc/histidine/polyglutamic acid complex according to the present invention.

Further, it is disclosed a food composition for preventing or alleviating pollinosis containing the the zinc/histidine/polyglutamic acid complex according to the present invention.

In another aspect, the present invention provides a food composition having anticancer activity and containing the zinc/histidine/polyglutamic acid complex according to the present invention.

Further, it is disclosed a pharmaceutical composition for preventing, alleviating or treating pollinosis containing the the zinc/histidine/polyglutamic acid complex according to the present invention.

In another aspect, the present invention provides an anticancer pharmaceutical composition containing the zinc/histidine/polyglutamic acid complex according to the present invention.

In another aspect of the present invention, the cancer includes at least one of Hodgkin's malignant lymphoma, breast cancer, glioblastoma and lung cancer.

### [Advantageous Effects]

The water-soluble zinc-amino acid complex provided by the present invention supplies zinc to humans or animals and plants in need thereof to prevent abnormal symptoms caused by lack of zinc, and these compounds can also be used for providing anticancer activity and alleviating the symptoms of pollinosis caused by pollen in the spring.

### [Brief Description of Drawings]

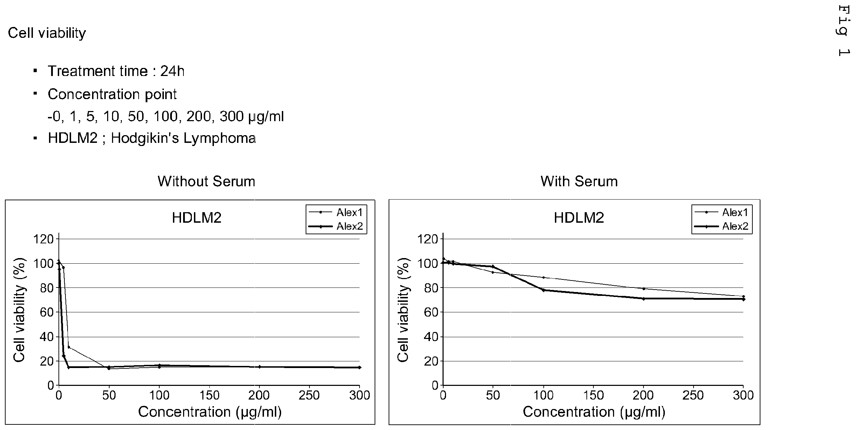

FIG. 1 shows the result of measurement of anticancer activity against Hodgkin's malignant lymphoma cells according to Experimental Example 3-1 of the present invention, wherein Alex1 is a zinc-histidine complex (Comparative Example 1), Alex2 is a zinc-histidine/polygamma-glutamic-acid complex (Example 1), and HDLM2 is a Hodgkin's malignant lymphoma cell.

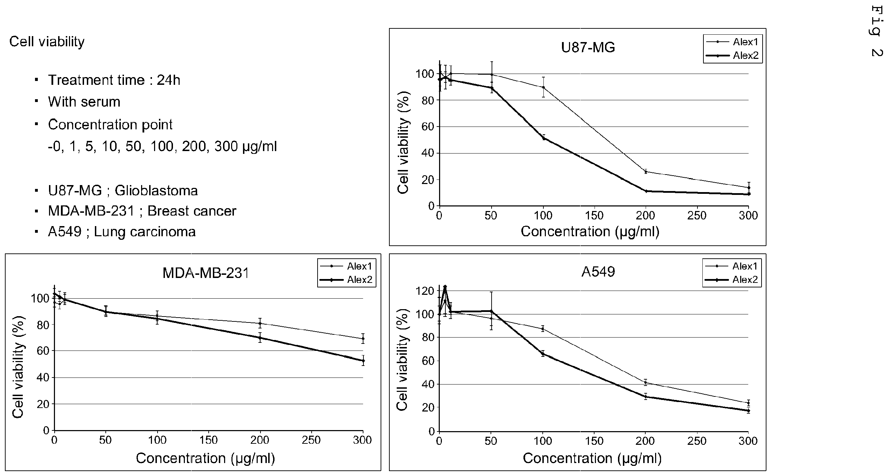

FIG. 2 shows the result of measurement of anticancer activity against breast cancer cells, glioblastoma in brain and lung carcinoma cells in the presence of serum according to Experimental Example 3-2 of the present invention, wherein Alex1 is a zinc-histidine complex (Comparative Example 1), Alex2 is a zinc-histidine-polygamma glutamic acid complex (Example 1), MDA-MB-231 is a breast cancer cell, U87-MG is a glioblastoma in the brain, and A549 is a lung carcinoma cell.

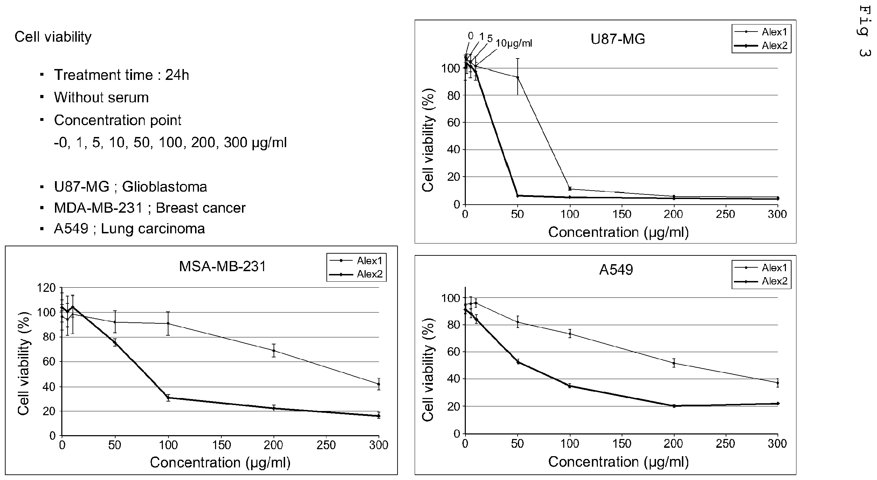

FIG. 3 shows the result of measurement of anticancer activity against breast cancer cells, glioblastoma in the brain and lung carcinoma cells in the absence of serum according to Experimental Example 3-3 of the present invention, wherein Alex1 is a zinc-histidine complex (Comparative Example 1), Alex2 is a zinc-histidine-polygamma glutamic acid complex (Example 1), MDA-MB-231 is a breast cancer cell, U87-MG is a glioblastoma in the brain, and A549 is a lung carcinoma cell.

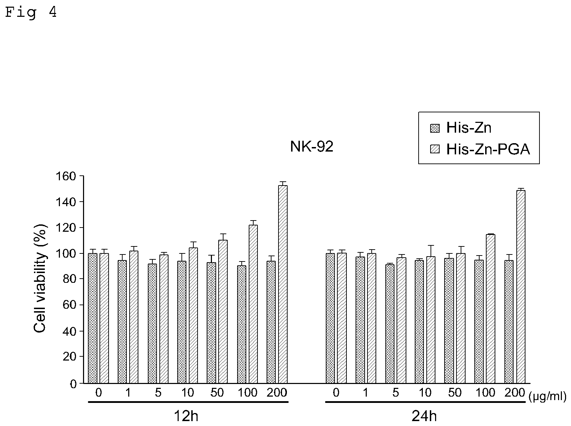

FIG. 4 shows the result of measurement of the activity of NK cells according to Experimental Example 3-4 of the present invention.

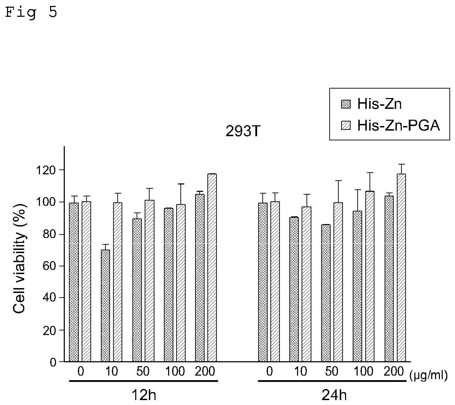

FIG. 5 shows the result of measurement of toxicity to normal cells according to Experimental Example 4 of the present invention.

### (Best Mode]

Unless the context clearly indicates otherwise, all numbers, figures and/or expressions that represent ingredients, reaction conditions, polymer compositions and amounts of mixtures used in the specification are approximations that reflect various uncertainties of measurement occurring inherently in obtaining these figures, among other things. For this reason, it should be understood that, in all cases, the term "about" should modify all numbers, figures and/or expressions. In addition, when numerical ranges are disclosed in the description, these ranges are continuous and include all numbers from the minimum to the maximum, including the maximum within the range, unless otherwise defined. Furthermore, when the range refers to an integer, it includes all integers from the minimum to the maximum, including the maximum within the range, unless otherwise defined.

It should be understood that, in the specification, when a range is referred to regarding a parameter, the parameter encompasses all figures including end points disclosed within the range. For example, the range of "5 to 10" includes figures of 5, 6, 7, 8, 9, and 10, as well as arbitrary sub-ranges, such as ranges of 6 to 10, 7 to 10, 6 to 9, and 7 to 9, and any figures, such as 5.5, 6.5, 7.5, 5.5 to 8.5 and 6.5 to 9, between appropriate integers that fall within the range. In addition, for example, the range of "10% to 30%" encompasses all integers that include numbers such as 10%, 11%, 12% and 13%, as well as 30%, and any sub-ranges, such as 10% to 15%, 12% to 18%, or 20% to 30%, as well as any numbers, such as 10.5%, 15.5% and 25.5%, between appropriate integers that fall within the range.

Hereinafter, the present invention will be described in detail.

In the present invention, a stable zinc/polyglutamic acid/histidine complex that is highly soluble in water is prepared by binding L-histidine and zinc to polyglutamic acid, and these complexes are used alone or are mixed with various adjuvants to produce capsule, injection or powder formulations. These substances were found to serve as a supply of zinc that is very well absorbed in the human body, and to have anticancer activity against cancer cells and alleviate pollinosis resulting from irritation of mucous membranes by pollen, and thus are intended for use in humans, animals and plants.

The synthesis of the zinc/polyglutamic acid/histidine complex is not disclosed in papers or patents, but the preparation of a complex of zinc and general amino acids is disclosed in papers and patents. Additionally, descriptions or uses associated with methods of preparing the polyglutamic acid-histidine-zinc complex are not disclosed in these prior art patents and papers.

Thus, a water-soluble zinc-containing polyglutamic acid-histidine complex, which is well absorbed by the human body, is prepared, and it can be seen from experiments that such a substance serves as a supply of zinc that has high absorbance efficiency and has anticancer activity against cancer cells and alleviates pollinosis resulting from irritation of mucous membranes by pollen, and thus a composition that can be used in humans, animals and plants is prepared and provided.

In one embodiment of the present invention, histidine, polygamma glutamic acid and zinc oxide are added to water, the resulting mixture is completely dissolved while refluxing for 2 to 10 hours, and is then distilled until a certain amount of water remains, and a solid is precipitated using various types of harmless alcohols such as ethanol, isopropanol and butanol and is then dried at room temperature for 12 to 36 hours to obtain the desired zinc/histidine/polyglutamic acid complex.

In one embodiment of the present invention, histidine, polygamma glutamic acid, and zinc acetate are added to water, the resulting mixture is completely dissolved while refluxing for 2 to 10 hours and then is distilled until a certain amount of water remains, and a solid is precipitated using various types of harmless alcohols, such as ethanol, isopropanol and butanol, and is then dried at room temperature for 12 to 36 hours to obtain the desired zinc/histidine/polyglutamic acid complex.

In one embodiment of the present invention, histidine, polygamma glutamic acid, and zinc carbonate are added to water, the resulting mixture is completely dissolved while refluxing for 2 to 10 hours and is then distilled until a certain amount of water remains, and a solid is precipitated using various types of harmless alcohols, such as ethanol, isopropanol and butanol and is then dried at room temperature for 12 to 36 hours to obtain the desired zinc/histidine/polyglutamic acid complex.

Hereinafter, various aspects of the present invention will be described.

It is diclosed a zinc/polyglutamic acid complex prepared from polygamma glutamic acid and a zinc supply.

According to the present invention there is provided a zinc/histidine/polyglutamic acid complex prepared from histidine, polygamma glutamic acid and a zinc supply.

According to the present invention, the complex is prepared from 30 to 70 parts by weight of the polygamma glutamic acid and 30 to 150 parts by weight of the zinc supply based on 200 parts by weight of the histidine.

In an aspect of the present invention, the zinc supply includes at least one of zinc oxide, zinc acetate and zinc carbonate.

In another aspect of the present invention, the complex contains zinc in an amount of 15% by weight or more based on the total weight of the complex.

In one aspect of the present invention, the complex is prepared by adding histidine, polygamma glutamic acid and a zinc supply to water, followed by reflux and then precipitation using a lower alcohol having 1 to 5 carbon atoms.

In another aspect, the present invention provides a method for preparing a zinc/histidine/polyglutamic acid complex, including:
(a) adding histidine, polygamma glutamic acid, and a zinc supply to water, followed by reflux;
(b) distilling the refluxed mixture; and
(c) adding a lower alcohol having 1 to 5 carbon atoms thereto to precipitate the complex.

According to the present invention, the step (a) includes adding 30 to 70 parts by weight of the polygamma glutamic acid and 30 to 150 parts by weight of the zinc supply, based on 200 parts by weight of the histidine, followed by reflux.

In another aspect of the present invention, the reflux is carried out for 30 minutes to 4 hours, and the lower alcohol having 1 to 5 carbon atoms includes at least one of isopropanol, butanol and ethanol.

In another aspect, the present invention provides a health food composition for supplying zinc containing the zinc/histidine/polyglutamic acid complex according to the present invention. Zinc deficiency causes symptoms such as reduced resistance to allergens such as pollen, as well as deterioration of immune function, erythema, hair loss, incrustation in pressed points, dandruff, pus discharge near body openings, itching, rough skin, excessive sebum, secondary bacterial infection, hyperkeratosis and excessive pigmentation. In particular, zinc deficiency increases the likelihood of pollinosis due to deterioration of immune function. Therefore, the food composition as a zinc supply can prevent or alleviate the diseases or symptoms described above, and in particular, can prevent or alleviate pollinosis caused by deterioration of immune function.

In another aspect, the present invention provides a food composition for preventing or alleviating pollinosis containing the zinc/histidine/polyglutamic acid complex according to one aspect of the present invention.

In another aspect, the present invention provides a food composition having anticancer activity and containing the zinc/histidine/polyglutamic acid complex according to one aspect of the present invention.

The food composition according to another aspect of the present invention contains the zinc/histidine/polyglutamic acid complex as an active ingredient. The active ingredient may be ingested as a food prepared in the form of a formulation such as a tablet, capsule, powder, granule, pill, liquid, suspension or the like, or may be ingested as a conventional food containing the same. The health food uses food as a raw material, unlike general drugs, thus having an advantage in that there are no side effects that may occur upon administration of the drug for a long time. The content of the active ingredient may be appropriately determined according to the purpose of use (prevention or amelioration), and the active ingredient may be present in a range of 0.1 to 90% by weight with respect to the total weight of the health food composition. However, in the case of long-term intake for health and hygiene purposes or for health control, the amount may be below the above range, or alternatively, the active ingredient may be used in an amount above the range, since there is no problem in terms of safety.

There is no particular limitation on the type of food. Examples of foods to which the active ingredient can be added include all health functional foods in the ordinary sense, including drinks, beverages, ionized beverages, dairy products, meat, sausage, bread, noodles, candy, snacks, gum, tea and vitamin complexes. The properties of the food are also not particularly limited, and may be solid, semi-solid, gel, liquid, powder or the like.

A health drink may be prepared using the health food composition. There is no particular limitation as to the ingredients of the health drink, other than the active ingredient. The health drink may contain, in addition to the active ingredient, natural carbohydrates or flavoring agents as additives commonly used in the preparation of beverages. The natural carbohydrates may include conventional sugars, such as monosaccharides (e.g. glucose, fructose, etc.), disaccharides (e.g. maltose, sucrose, etc.) and polysaccharides (e.g., dextrin, cyclodextrin, etc.), as well as sugar alcohols such as xylitol, sorbitol and erythritol. Further, useful flavoring agents may include natural flavoring agents (thaumatin), stevia extracts (rebaudioside A, glycyrrhizin, etc.) and synthetic flavoring agents (saccharin, aspartame, etc.). The natural carbohydrate may be present in an amount of about 1 to about 20 g, preferably about 5 to about 10 g, with respect to 100 mL of the composition of the present invention.

Further, in addition to the active ingredient, the food composition of the present invention may contain other nutrients, vitamins, minerals (electrolytes), flavors such as synthetic or natural flavors, colorants and fillers (cheese, chocolate, etc.), pectic acids and salts thereof, alginic acids and salts thereof, organic acids, protective colloidal thickeners, pH-adjusting agents, stabilizers, preservatives, glycerin, alcohol, carbonic acid used in carbonated beverages, and the like. In addition, it may contain flesh for the production of natural fruit juices, fruit juice beverages and vegetable beverages. These components may be used independently or in combination. Although the proportion of these additives is not so important, the content of these additives is generally selected within the range of 0.1 to 20% by weight in the health food composition of the present invention.

In another aspect, the present invention provides a pharmaceutical composition for preventing, alleviating or treating pollinosis containing the zinc/histidine/polyglutamic acid complex according to the present invention.

In another aspect, the present invention provides an anticancer pharmaceutical composition containing the zinc/histidine/polyglutamic acid complex according to the present invention.

In another aspect of the present invention, the cancer includes at least one of Hodgkin's malignant lymphoma, breast cancer, glioblastoma and lung cancer.

The pharmaceutical composition according to another aspect of the present invention is prepared as a formulation for oral administration, such as a tablet, capsule, troche, solution or suspension, prepared by adding conventional pharmaceutically acceptable non-toxic carriers, adjuvants, excipients and the like. Examples of excipients that can be used in the complex composition of the present invention may include sweeteners, binders, solubilizers, solubilization aids, wetting agents, emulsifiers, isotonic agents, adsorbents, disintegrants, antioxidants, preservatives, lubricants, fillers, fragrances and the like. For example, the excipient may include lactose, dextrose, sucrose, mannitol, sorbitol, cellulose, glycine, silica, talc, stearic acid, stearin, magnesium stearate, magnesium aluminum silicate, starch, gelatin, rubber tragacanth, alginic acid, sodium alginate, methylcellulose, sodium carboxymethylcellulose, agar, water, ethanol, polyethylene glycol, polyvinylpyrrolidone, sodium chloride, calcium chloride, orange essence, strawberry essence, vanilla flavor, and the like.

In addition, the dosage of the complex of the present invention or a pharmaceutical composition containing the same administered to the human body may vary depending on the patient's age, weight, gender, dosage form, health condition, and severity of disease, based on an adult patient weighing 70 kg. In general, the dosage is 0.01 mg to 5,000 mg per day, and may be administered once a day, or may be divided and administered in several portions throughout the day at regular intervals.

### [Best mode]

The present invention as described above will be described in more detail based on the following examples, but the present invention is not limited to these examples. Examples 5-7 are not forming part of the invention.

### [Example]

### Example 1. Synthesis of zinc-histidine-polygamma glutamic acid complex (Alex2) using zinc oxide

200 g of histidine, 50 g of polygamma glutamic acid and 49.8 g of zinc oxide were added to 1,000 mL of water, followed by complete dissolution while refluxing for 2 hours and then distillation of remaining water. 1,000 mL of ethanol was added to the residue to obtain a solid, and the solid was filtered and dried at room temperature for 24 hours to obtain 270 g of a white powdery solid.

¹H-NMR (400 MHz, D₂O) δ7.61 (s, 1H), 6.90 (s, 1H), 3.86 (m, 1H), 3.07 (m, 2H), 2.22 (m, 0.5H), 1.82 (m, 0.5H); atomic absorption spectroscopy (AAS) zinc content: 15.4%.

### Example 2. Synthesis of zinc-histidine-polygamma glutamic acid complex using zinc acetate

200 g of histidine, 50 g of polygamma glutamic acid and 134.3 g of zinc acetate were added to 1,000 mL of water, followed by complete dissolution while refluxing for 2 hours and then distillation of remaining water. 1,000 mL of ethanol was added to the residue to obtain a solid, and the solid was filtered and dried at room temperature for 24 hours to obtain 260 g of a white powdery solid.

¹H-NMR (400 MHz, D₂O) δ7.61 (s, 1H), 6.90 (s, 1H), 3.86 (m, 1H), 3.07 (m, 2H), 2.22 (m, 0.5H), 1.82 (m, 0.5H); Atomic absorption spectroscopy (AAS) zinc content 15.9%.

### Example 4. Synthesis of zinc-histidine-polygamma glutamic acid complex using zinc acetate

200 g of histidine, 50 g of polygamma glutamic acid and 76.7 g of zinc carbonate were added to 1,000 mL of water, followed by complete dissolution while refluxing for 2 hours and then distillation of the remaining water. 1,000 mL of ethanol was added to the residue to obtain a solid, and the solid was filtered and dried at room temperature for 24 hours to obtain 250 g of a white powdery solid.

¹H-NMR (400 MHz, D₂O) δ7.61 (s, 1H), 6.90 (s, 1H), 3.86 (m, 1H), 3.07 (m, 2H), 2.22 (m, 0.5H), 1.82 (m, 0.5H); Atomic absorption spectroscopy (AAS) zinc content 16.9%.

### Example 5. Synthesis of zinc-polygamma glutamic acid complex using zinc oxide (not forming part of the invention)

10 g of polygamma glutamic acid and 3.54 g of zinc oxide were added to 200 mL of water, followed by complete dissolution while refluxing for 2 hours and then distillation of remaining water. 1,000 mL of ethanol was added to the residue to obtain a solid, and the solid was filtered and dried at room temperature for 24 hours to obtain 13 g of a white powdery solid.

¹H-NMR (400 MHz, DMSO-d₆ + D₂O) δ8.20 (s, 1H), 4.10 (s, 1H), 2.20 (bs, 2H), 1.98 (bs, 1H), 1.89 (bs, 1H).

### Example 6. Synthesis of zinc-polygamma glutamic acid complex using zinc acetate (not forming part of the invention)

10 g of polygamma glutamic acid and 9.54 g of zinc acetate were added to 200 mL of water, followed by reflux for 2 hours. The resulting solid was filtered and dried at room temperature for 24 hours to obtain 11 g of a white powdery solid.

¹H-NMR (400 MHz, DMSO-d₆ + D₂O) δ 8.20 (s, 1H), 4.10 (s, 1H), 2.20 (bs, 2H), 1.98 (bs, 1H), 1.89 (bs, 1H).

### Example 7. Synthesis of zinc-polygamma glutamic acid complex using zinc carbonate (not forming part of the invention)

10 g of polygamma glutamic acid and 5.45 g of zinc carbonate were added to 200 mL of water, followed by reflux for 2 hours. The resulting solid was filtered and dried at room temperature for 24 hours to obtain 10 g of a white powdery solid.

¹H-NMR (400 MHz, DMSO-d₆ + D₂O) δ8.20 (s, 1H), 4.10 (s, 1H), 2.20 (bs, 2H), 1.98 (bs, ¹H), 1.89 (bs, 1H).

### Comparative Example 1. Synthesis of zinc histidine complex (Alex1)

250 g of histidine and 65.6 g of zinc acetate were added to 2,000 mL of water, followed by reflux for 2 hours. 1,500 mL of water was distilled and 1,000 mL of ethanol was added to the residue to precipitate a solid. The solid was filtered and dried at room temperature for 24 hours to obtain 298.0 g of a zinc histidine complex.

Yield 99%; ¹H-NMR (400 MHz, DMSO-d₆ + D₂O) δ7.65 (s, 1H), 6.96 (s, 1H), 3.89 (m, 1H), 3.10 (m, 2H); Atomic absorption spectroscopy (AAS) zinc content 14.5%.

### [Experimental Example]

The following experiment was conducted on Examples and Comparative Examples prepared above.

### Experimental Example 1. Water solubility of zinc-histidine-polyglutamic acid complex (Alex2)

When 1 g of zinc oxide was added to 25 mL of water at room temperature (about 25°C, 1 atmosphere), the solution remains almost insoluble, whereas the zinc-histidine-polyglutamic acid complexes of Examples 1 to 4 were dissolved under the same conditions immediately after addition to water.

The zinc-polyglutamic acid complexes of Examples 5 to 7 were less soluble than the zinc-histidine-polyglutamic acid complexes under the same conditions, but were relatively well dissolved.

### Experimental Example 2. Zinc content of zinc-histidine-polyglutamic acid complex

The zinc content of each of the zinc-histidine complex of Comparative Example 1 and the zinc-histidine-polygamma glutamic acid complex of Example 1 was investigated using atomic absorption spectroscopy (AAS). The result showed that the zinc content of Comparative Example 1 was 14.5%, whereas the zinc content of Example 1 was 15.4% (weight ratio). In addition, the zinc content of the zinc-polyglutamic acid complex of Example 5 was 16.6% (weight ratio).

### Experimental Example 3. Anticancer activity of zinc-histidine-polyglutamic acid complex

### Experimental Example 3-1. Measurement of anticancer activity against Hodgkin's malignant lymphoma

Anticancer activity against Hodgkin's malignant lymphoma cells was measured on Example 1 and Comparative Example 1.

When HDLM2 (Hodgkin's malignant lymphoma) cells obtained from pleural effusion of a 74-year-old male having crystalline sclerosing Hodgkin's disease stage 4 were treated in the presence and the absence of serum with the complex of Example 1 and the complex of Comparative Example 1 at concentrations of 0, 1, 5, 10, 50, 100, 200 and 300 µg/ml for 24 hours, cell viability of the cells was measured using an ELISA reader through an MTT assay.

The experimental results were shown in FIG. 1.

### Experimental Example 3-2. Measurement of anticancer activity against breast cancer, glioblastoma and lung cancer cells in the presence of serum

The anticancer activity against each of breast cancer, glioblastoma and lung cancer cells of Example 1 and Comparative Example 1 was measured in the presence of serum.

The breast cancer cells used herein were human breast cancer cells, MDA-MB-231.

The glioblastoma cells used herein were U87-MG glial cancer cells obtained from a 44-year-old woman.

The lung cancer cells used herein were A549 adenocarcinoma cells obtained from a 58-year-old male.

When the cells were treated in the presence of serum with Example 1 and Comparative Example 1 at concentrations of 0, 1, 5, 10, 50, 100, 200 and 300 µg/ml for 24 hours, cell viability of the cells was measured.

The experimental results are shown in FIG. 2.

### Experimental Example 3-3. Measurement of anticancer activity against breast cancer, glioblastoma and lung cancer cells in the absence of serum

The anticancer activity against each of breast cancer, glioblastoma and lung cancer cells of Example 1 and Comparative Example 1 was measured in the absence of serum.

The experimental results are shown in FIG. 3.

### Experimental Example 3-4. Measurement of activity of NK cells as immune cells

The activity of NK cells was measured with regard to Example 1 and Comparative Example 1. NK cells are known to play an important role in the elimination of cancer cells.

NK-92 cells derived from a patient suffering from NK cell lymphoma as the NK cell line were treated with Example 1 and Comparative Example 1, and the activity of NK cells was measured.

The experimental results are shown in FIG. 4. As can be seen from FIG. 4, the histidine/zinc/polyglutamic acid complex has superior ability to inhibit the progression of cancer by improving the proliferation of NK cells, which plays an important role in the removal of cancer cells, compared to Comparative Example 1.

### Experimental Example 4. Toxicity test of Example 1 and Comparative Example 1 on normal cells

Toxicity of Example 1 and Comparative Example 1 was measured in human embryonic kidney cell line cells, which are normal cells. Toxicity was measured after treatment of 293T cells derived from human kidney cells with Example 1 and Comparative Example 1.

The experimental results are shown in FIG. 5. As can be seen from FIG. 5, the histidine/zinc/polyglutamic acid complex of Example 1 decreased cytotoxicity in normal cells compared to Comparative Example 1. Therefore, it can be seen that the complex of Example 1 specifically exhibits cytotoxicity on cancer cells compared to the complex of Comparative Example 1.

As can be seen from the above experimental examples, the novel zinc-histidine-polyglutamic acid complex or the zinc-polyglutamic acid complex according to the present invention, which is produced as a capsule or injection formulation, serves as a supply of zinc essential for the human body and has anticancer activity against cancer cells and an effect of alleviating pollinosis resulting from irritation of mucous membranes by pollen. As a result, a composition that can be used in humans, animals and plants was prepared and provided.

### [Formulation Example]

Meanwhile, the zinc/polyglutamic acid complex (not according to the invention) or the zinc/histidine/polyglutamic acid complex according to the present invention can be formulated in various forms depending on the purpose. The following exemplifies methods of obtaining some formulations containing the compound of Example 5 among the zinc/polyglutamic acid complexes not according to the present invention and the compound of Example 1 among the zinc/histidine/polyglutamic acid complexes.

### Formulation Example 1. Tablet (direct pressurization)

5.0 mg of the compound of Example 1 was sieved and mixed with 14.1 mg of lactose, 0.8 mg of crospovidone USNF and 0.1 mg of magnesium stearate, and the resulting mixture was pressed into tablets.

### Formulation Example 2. Tablet (Wet granulation)

5.0 mg of the compound of Example 1 was sieved and mixed with 16.0 mg of lactose and 4.0 mg of starch. 0.3 mg of Polysorbate 80 was dissolved in pure water, and an appropriate amount of the resulting solution was added to the resulting mixture, followed by granulation. The granules were dried, sieved and mixed with 2.7 mg of colloidal silicon dioxide and 2.0 mg of magnesium stearate. The granules were pressed into tablets.

### Formulation Example 3. Powders and capsules

5.0 mg of the compound of Example 1 was sieved and was then mixed with 14.8 mg of lactose, 10.0 mg of polyvinylpyrrolidone and 0.2 mg of magnesium stearate. Hard No. 5 gelatin capsules were filled with the resulting mixture.

### Formulation Example 4. Injection

Injections were prepared by incorporating 100 mg of the compound of Example 1 as well as 180 mg of mannitol, 26 mg of Na₂HPO₄/H₂O and 2,974 mg of distilled water.

### Formulation Example 5. Tablet (direct pressurization)

5.0 mg of the compound of Example 5 was sieved and mixed with 14.1 mg of lactose, 0.8 mg of crospovidone USNF and 0.1 mg of magnesium stearate, and the resulting mixture was pressed into tablets.

### Formulation Example 6. Tablet (Wet granulation)

5.0 mg of the compound of Example 5 was sieved and mixed with 16.0 mg of lactose and 4.0 mg of starch. 0.3 mg of Polysorbate 80 was dissolved in pure water, and an appropriate amount of the resulting solution was added to the resulting mixture, followed by granulation. The granules were dried, sieved and mixed with 2.7 mg of colloidal silicon dioxide and 2.0 mg of magnesium stearate. The granules were pressed into tablets.

### Formulation Example 7. Powders and capsules

5.0 mg of the compound of Example 5 was sieved and then mixed with 14.8 mg of lactose, 10.0 mg of polyvinylpyrrolidone and 0.2 mg of magnesium stearate. Hard No. 5 gelatin capsules were filled with the resulting mixture.

### Formulation Example 8. Injection

Injections were prepared by incorporating 100 mg of the compound of Example 5 as well as 180 mg of mannitol, 26 mg of Na₂HPO₄/H₂O and 2,974 mg of distilled water.

## Claims

1. A zinc/histidine/polyglutamic acid complex prepared from histidine, polygamma glutamic acid and a zinc supply, wherein the histidine is L-histidine, and wherein the complex is prepared from 30 to 70 parts by weight of the polygamma glutamic acid and 30 to 150 parts by weight of the zinc supply based on 200 parts by weight of the histidine.

2. The zinc/histidine/polyglutamic acid complex according to claim 1, wherein the zinc supply comprises at least one of zinc oxide, zinc acetate and zinc carbonate.

3. The zinc/histidine/polyglutamic acid complex according to claim 1 or 2, wherein the complex comprises zinc in an amount of 15% by weight or more based on the total weight of the complex.

4. A method for preparing the zinc/histidine/polyglutamic acid complex according to any one of claims 1 to 3, comprising:
(a) adding L-histidine, polygamma glutamic acid and a zinc supply to water, followed by reflux;
(b) distilling the refluxed mixture; and
(c) adding a lower alcohol having 1 to 5 carbon atoms thereto to precipitate the complex,
wherein the step (a) comprises adding 30 to 70 parts by weight of the polygamma glutamic acid and 30 to 150 parts by weight of the zinc supply, based on 200 parts by weight of the histidine, followed by reflux.

5. The method according to claim 4, wherein the reflux is carried out for 30 minutes to 4 hours, and the lower alcohol having 1 to 5 carbon atoms comprises at least one of isopropanol, butanol and ethanol.

6. A health food composition for supplying zinc comprising the zinc/histidine/polyglutamic acid complex according to any one of claims 1 to 3.

7. A food composition having anticancer activity and comprising the zinc/histidine/polyglutamic acid complex according to any one of claims 1 to 3.

8. An anticancer pharmaceutical composition comprising the zinc/histidine/polyglutamic acid complex according to any one of claims 1 to 3.

9. The anticancer pharmaceutical composition according to claim 8, wherein the cancer comprises at least one of Hodgkin's malignant lymphoma, breast cancer, glioblastoma and lung cancer.

## Patentansprüche

1. Zink/Histidin/Polyglutaminsäure-Komplex, hergestellt aus Histidin, Polygamma-Glutaminsäure und einer Zinkquelle, wobei das Histidin L-Histidin ist, und wobei der Komplex, auf der Grundlage von 200 Gewichtsteilen Histidin, mit 30 bis 70 Gewichtsteilen der Polygamma-Glutaminsäure und 30 bis 150 Gewichtsteilen der Zinkquelle hergestellt ist.

2. Zink/Histidin/Polyglutaminsäure-Komplex nach Anspruch 1, wobei die Zinkquelle wenigstens eines von Zinkoxid, Zinkacetat und Zinkcarbonat enthält.

3. Zink/Histidin/Polyglutaminsäure-Komplex nach Anspruch 1 oder 2, wobei der Komplex, bezogen auf das Gesamtgewicht des Komplexes, Zink in einer Menge von 15 Gew.-% oder mehr enthält.

4. Verfahren zur Herstellung des Zink/Histidin/Polyglutaminsäure-Komplexes nach einem der Ansprüche 1 bis 3, umfassend:
(a) Zugabe von L-Histidin, Polygamma-Glutaminsäure und einer Zinkquelle zu Wasser, gefolgt von Rückfluss,
(b) Destillieren der unter Rückfluss stehenden Mischung und
(c) Zugabe eines niederen Alkohols mit 1 bis 5 Kohlenstoffatomen dazu, um den Komplex auszufällen,
wobei der Schritt (a) auf der Grundlage von 200 Gewichtsteilen Histidin die Zugabe von 30 bis 70 Gewichtsteilen der Polygamma-Glutaminsäure und von 30 bis 150 Gewichtsteilen der Zinkquelle umfasst, gefolgt von Rückfluss.

5. Verfahren nach Anspruch 4, wobei der Rückfluss 30 Minuten bis 4 Stunden lang durchgeführt wird und der niedere Alkohol mit 1 bis 5 Kohlenstoffatomen wenigstens einen von Isopropanol, Butanol und Ethanol enthält.

6. Gesundheitsfördernde Nahrungsmittelzusammensetzung zur Versorgung mit Zink, die den Zink/Histidin/Polyglutaminsäure-Komplex nach einem der Ansprüche 1 bis 3 enthält.

7. Nahrungsmittelzusammensetzung mit Anti-Krebs-Aktivität, die den Zink/Histidin/Polyglutaminsäure-Komplex nach einem der Ansprüche 1 bis 3 enthält.

8. Pharmazeutische Anti-Krebs-Zusammensetzung, die den Zink/Histidin/Polyglutaminsäure-Komplex nach einem der Ansprüche 1 bis 3 enthält.

9. Pharmazeutische Anti-Krebs-Zusammensetzung nach Anspruch 8, wobei der Krebs wenigstens einen von Hodgkin's malignem Lymphom, Brustkrebs, Glioblastom und Lungenkrebs umfasst.

## Revendications

1. Complexe de zinc/histidine/acide polyglutamique préparé à partir d'histidine, d'acide polygamma-glutamique et d'un apport en zinc, dans lequel l'histidine est la L-histidine, et dans lequel le complexe est préparé à partir de 30 à 70 parties en poids de l'acide polygamma-glutamique et de 30 à 150 parties en poids de l'apport en zinc, sur la base de 200 parties en poids de l'histidine.

2. Complexe de zinc/histidine/acide polyglutamique selon la revendication 1, dans lequel l'apport en zinc comprend au moins un élément parmi l'oxyde de zinc, l'acétate de zinc et le carbonate de zinc.

3. Complexe de zinc/histidine/acide polyglutamique selon la revendication 1 ou 2, dans lequel le complexe comprend du zinc en une quantité de 15 % en poids ou plus, sur la base du poids total du complexe.

4. Procédé de préparation du complexe de zinc/histidine/acide polyglutamique selon l'une quelconque des revendications 1 à 3, comprenant les étapes consistant à :
(a) ajouter de la L-histidine, de l'acide polygamma-glutamique et un apport en zinc dans de l'eau, suivi par un reflux ;
(b) distiller le mélange à reflux ; et
(c) ajouter un alcool inférieur présentant 1 à 5 atomes de carbone pour précipiter le complexe,
dans lequel l'étape (a) comprend l'ajout de 30 à 70 parties en poids de l'acide polygamma-glutamique et de 30 à 150 parties en poids de l'apport en zinc, sur la base de 200 parties en poids de l'histidine, suivi d'un reflux.

5. Procédé selon la revendication 4, dans lequel le reflux est effectué pendant 30 minutes à 4 heures, et l'alcool inférieur présentant 1 à 5 atomes de carbone comprend au moins un composé parmi l'isopropanol, le butanol et l'éthanol.

6. Composition alimentaire diététique permettant un apport en zinc, comprenant le complexe de zinc/histidine/acide polyglutamique selon l'une quelconque des revendications 1 à 3.

7. Composition alimentaire présentant une activité anticancéreuse et comprenant le complexe de zinc/histidine/acide polyglutamique selon l'une quelconque des revendications 1 à 3.

8. Composition pharmaceutique anticancéreuse comprenant le complexe de zinc/histidine/acide polyglutamique selon l'une quelconque des revendications 1 à 3.

9. Composition pharmaceutique anticancéreuse selon la revendication 8, dans laquelle le cancer comprend au moins un cancer parmi le lymphome malin de Hodgkin, le cancer du sein, le glioblastome et le cancer du poumon.
